Europäisches Patentamt
European Patent Office
Office européen des brevets

(11) Publication number: **0 004 132**
**A1**

# EUROPEAN PATENT APPLICATION

(21) Application number: 79300218.9

(22) Date of filing: 14.02.79

(51) Int. Cl.²: C 07 D 487/04
A 61 K 31/40
//(C07D487/04, 209/00, 205/00)

(30) Priority: 04.03.78 GB 869078
04.03.78 GB 869178

(43) Date of publication of application:
19.09.79 Bulletin 79/19

(84) Designated contracting states:
BE CH DE FR GB IT NL SE

(71) Applicant: BEECHAM GROUP LIMITED
Beecham House Great West Road
Brentford, Middlesex(GB)

(72) Inventor: Corbett, David Francis
Dingle Bank Pilgrims Way West Humble
Dorking Surrey(GB)

(74) Representative: Cole, William Gwyn et al,
Beecham Pharmaceuticals Yew Tree Bottom Road
Epsom Surrey KT18 5XQ(GB)

BAD ORIGINAL

(54) **Esters of beta-lactam antibiotics, a process for their preparation and their use in pharmaceutical compositions.**

(57) The compounds of the formulae (V) - (X):

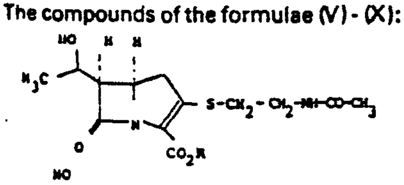
(V)

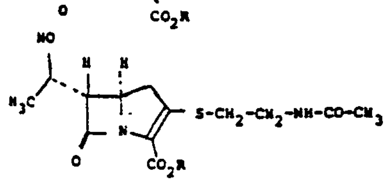
(VI)

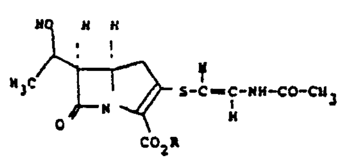
(VII)

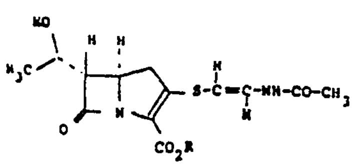
(VIII)

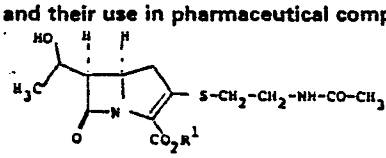
(XI)

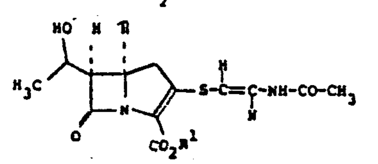
(X)

wherein R is a group of the sub-formula (a), (b) or (c):

$-CHR_1-O-CO-R_2$ (a)

$-CHR_1-O-CO-OR_2$ (b)

Artwork s 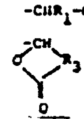 (c)

where $R_1$ is a hydrogen atom or a methyl or ethyl group; $R_2$ is a lower alkyl, lower-alkylaryl or aryl group; and $R_3$ is a $-CHR_4-CHR_4$ or $CR_5=CR_5$ group where $R_4$ is a hydrogen atom or a methyl group and $R_5$ is a hydrogen atom, a methyl group or $R_4$ and $R_5$ together with the carbon atoms to which they are attached form the residue of a phenyl group or of a phenyl group substituted by one or two lower alkyl or lower alkoxyl groups of fluorine, chlorine or bromine atoms; and $R^1$ is a lower alkyl, lower alkenyl, lower alkynyl or aralkyl group; are antibacterial agents. Their preparation and compositions are described.



Croydon Printing Company Ltd.

# Esters of β-Lactam antibiotics, a process for their preparation and their use in pharmaceutical compositions

British Patent Application No. 9366/77 - 32836/77 (see also Belgian Patent No. 864570 ) disclosed inter alia that the compounds of the formula (I) - (IV):

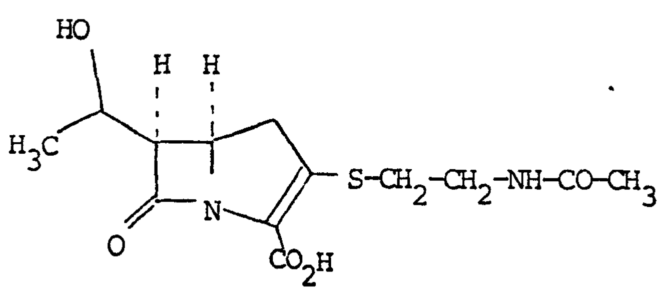

(I)

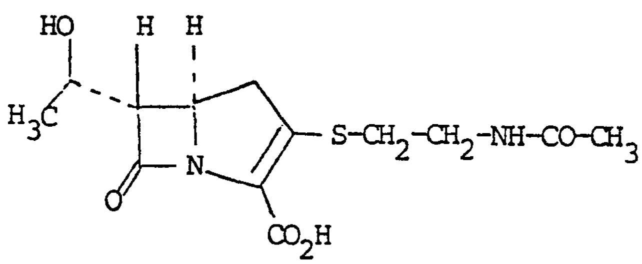

(II)

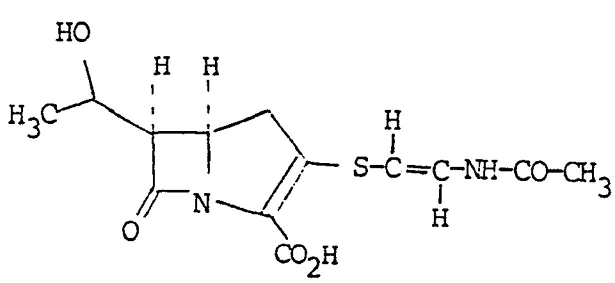

(III)

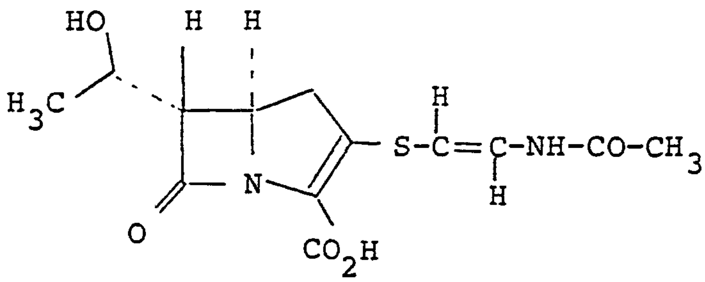

(IV)

and their pharmaceutically acceptable salts are useful antibacterial compounds. It has now been found that certain esters of such compounds have favoured antibacterial activity that allows them to be used in the treatment of infections due to gram-positive and gram-negative bacteria such as *Staphylococcus aureus* and *Proteus mirabilis*.

The present invention provides the compounds of the formulae (V)-(X):

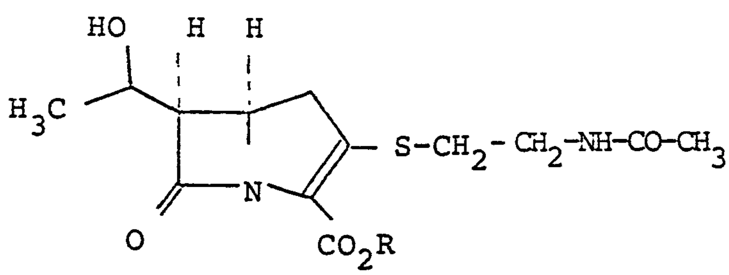

(V)

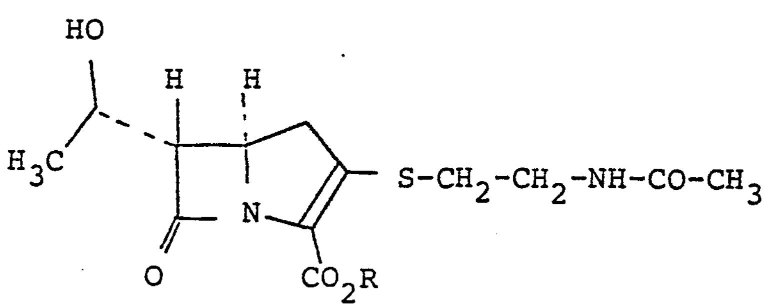

(VI)

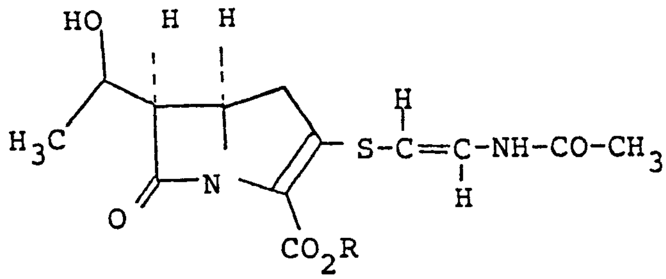

(VII)

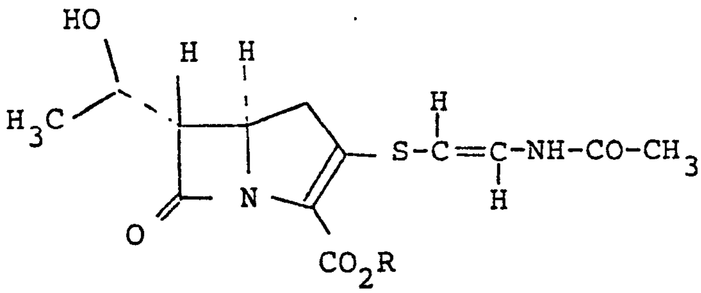

(VIII)

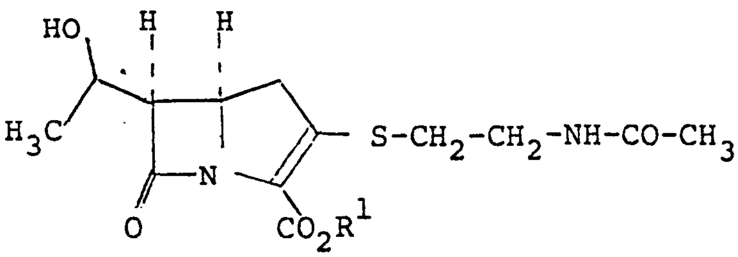

(IX)

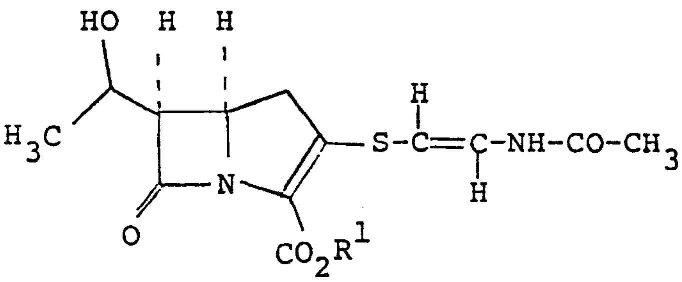

(X)

wherein R is a group of the sub-formula (a), (b) or (c):

$-CHR_1-O-CO-R_2$ (a)

$-CHR_1-O-CO-OR_2$ (b)

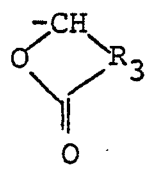 (c)

where $R_1$ is a hydrogen atom or a methyl or ethyl group; $R_2$ is a lower alkyl, lower-alkylaryl or aryl group; and $R_3$ is a $-CHR_4-CHR_4$ or $-CR_5=CR_5$ group where $R_4$ is a hydrogen atom or a methyl group and $R_5$ is a hydrogen atom, a methyl group or $R_4$ and $R_5$ together with the carbon atoms to which they are attached form the residue of a phenyl group or of a phenyl group substituted by one or two lower alkyl or lower alkoxyl groups of fluorine, chlorine or bromine atoms; and $R^1$ is a lower alkyl, lower alkenyl, lower alkynyl or aralkyl group.

When used herein the term "lower" means a group of up to 4 carbon atoms. When used herein the term "aryl" means phenyl or phenyl substituted by one or two lower alkyl or lower alkoxyl groups or fluorine, chlorine or bromine atoms. When used herein the term "aralkyl" means a lower alkyl group substituted by a phenyl group or by a phenyl group substituted by a nitro, lower alkyl or lower alkoxyl group or by a fluorine, chlorine or bromine atom.

More suitably $R_1$ is a hydrogen atom or a methyl group. Most suitably $R_3$ represents the residue of a phenyl ring.

Values of R particularly worthy of mention are acetoxymethyl, pivaloyloxymethyl, α-ethoxycarbonyloxyethyl and phthalidyl.

A preferred value for R is phthalidyl.

Values of $R^1$ particularly worthy of mention are the benzyl and substituted benzyl groups. Other suitable values for $R^1$ include the methyl, ethyl, propyl, allyl, propargyl and phenylethyl groups.

A preferred value for $R^1$ is the p-nitrobenzyl group.

Preferred compounds of this invention are esters of the cis-compounds of the formulae (I) and (III).

The esters of this invention may be used to treat bacterial infections. Thus in a further aspect this invention provides a pharmaceutical composition which comprises a compound of this invention and a pharmaceutically acceptable carrier.

Most suitably, the compositions of this invention are used to treat infections due to gram positive organisms such as Staphylococcus aureus.

The compositions of this invention will generally contain from 50mg to 500mg of a compound of this invention and may be administered once or more times a day so that the total daily dose (for a 70kg adult) is in the range 200mg to 2000mg.

The compositions of this invention may be administered orally or parenterally.

The compositions of this invention may also be formulated for intra mammary administration to cattle for the treatment of mastitis.

The compositions of this invention may be formulated in conventional manner, for example as for amoxycillin, thienamycin or the like.

Those compounds of this invention having these cis-configuration about the β-lactam are preferred since they tend to possess more potent antibacterial activity than the corresponding trans compound (as judged by in-vitro methods).

The present invention also provides a process for the preparation of a compound of this invention which comprises the reaction of a salt of a compound of the formula (I), (II), (III) or (IV) with a compound of the formula (XI), (XII), (XIII) or (XIV):

$Y-CHR_1-O-CO-R_2$ (XI)

$Y-CHR_1-O-CO-OR_2$ (XII)

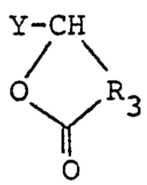

(XIII)

$Y-R^1$ (XIV)

wherein $R_1$, $R_2$ and $R_3$ are as defined in relation to sub-formulae (a), (b) and (c), $R^1$ is as defined in relation to formulae (IX) and (X) and Y is a group readily displaceable by a nucleophile.

Most suitably the salt employed is an alkali metal salt such as the sodium salt.

Most suitably Y is a chlorine, bromine or iodine atom.

The esterification reaction is normally performed in a polar organic solvent such as dimethylformamide or its equivalent. Generally the reaction is performed at a non-extreme temperature such as 0-60°C and may conveniently be carried out at ambient temperature.

The desired ester may be purified chromatographically if desired.

The compounds of this invention are not overtly toxic at the therapeutic dose.

The following examples illustrate the invention:

Example 1

Pthalidyl (5R,6S)-3-[(E)-2-acetamidoethenylthio]-6-[(S)-1-hydroxy-ethyl]-7-oxo-1-azabicyclo[3.2.0]hept-2-ene-2-carboxylate

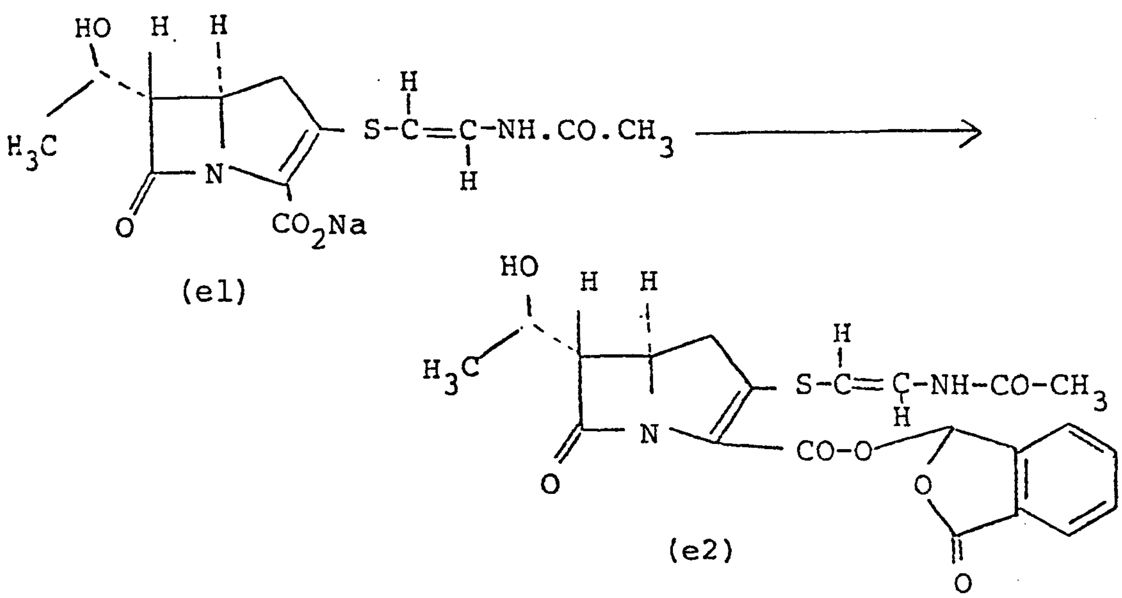

(e1)

(e2)

The sodium salt (e2) (0.312g of material approximately 70% pure) was stirred with bromophthalide (0.199g) in dimethylformamide (6ml) at room temperature for 1.5 hours. The mixture was concentrated in vacuo and the residue immediately chromatographed on silica gel (30g) eluting with chloroform/ethanol mixtures (starting with $CHCl_3$ grading to $CHCl_3$ : $C_2H_5OH$, 4:1). Fractions containing the product were combined and evaporated in vacuo. Further evaporation from toluene gave the phthalidyl ester (e2) as a pale yellow solid (0.15g).

$\lambda$ max ($C_2H_5OH$): 335 and 231 nm.

$\nu$ max ($CHCl_3$) : 1780, 1700 and 1625 $nm^{-1}$

the n.m.r. ($CDCl_3/CD_3SOCD_3$) showed singlets at $\delta$7.38 and $\delta$7.44 indicating that the ester was a mixture (about 1:1) of the two phthalidyl epimers.

Example 2

Phthalidyl (5R,6R)-3-[(E)-2-acetamidoethenylthio]-6-[(S)-1-hydroxyethyl]-7-oxo-1-azabicyclo[3.2.0]hept-2-ene-2-carboxylate

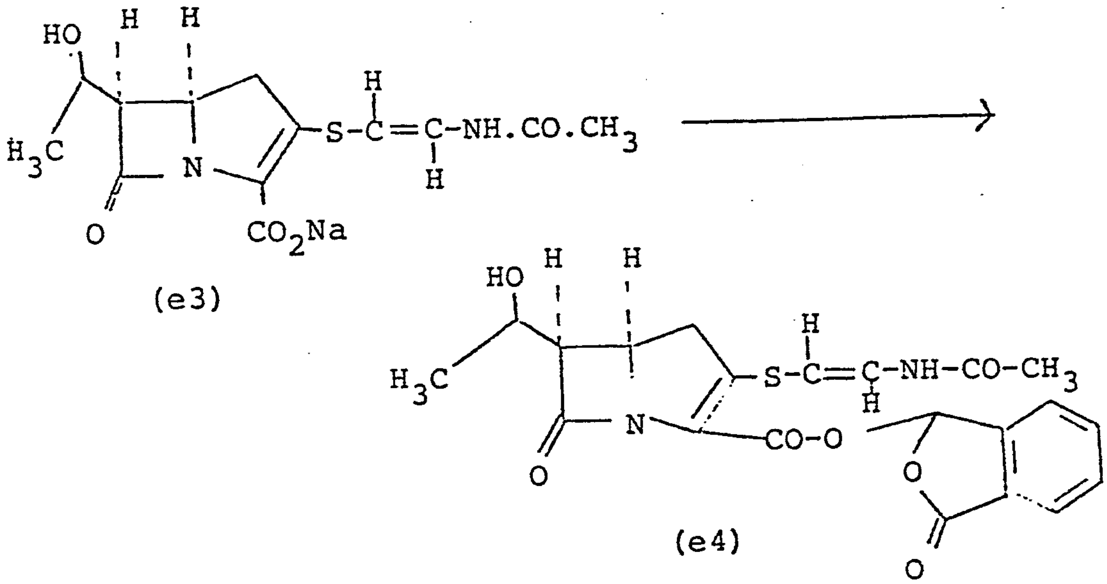

(e3)

(e4)

The sodium salt (e3) (0.179g) was treated with bromophthalide (0.115g) in dimethylformamide (4ml) as described in Example 1. The resulting ester (e4) was obtained as a cream solid (0.088g).

$\lambda$ max ($C_2H_5OH$) : 333 and 231 nm.

$\nu$ max (KBr) : 1775, 1700 and 1620 $nm^{-1}$

The n.m.r. indicated that the solid was a mixture of the two phthalidyl isomers.

The product of this Example had the following MIC's:

| Organism | | MIC (μg/ml) |
|---|---|---|
| *Staphylococcus aureus* | Russell | 0.4 |
| *Staphylococcus aureus* | Oxford | < 0.4 |
| *Staphylococcus* 1517 | | 0.4 |

Example 3

p-Nitrobenzyl (5R,6R)-3-(2-acetamidoethylthio-6-[(S)-1-hydroxyethyl)-7-oxo-1-azabicyclo[3.2.0]hept-2-ene-2-carboxylate

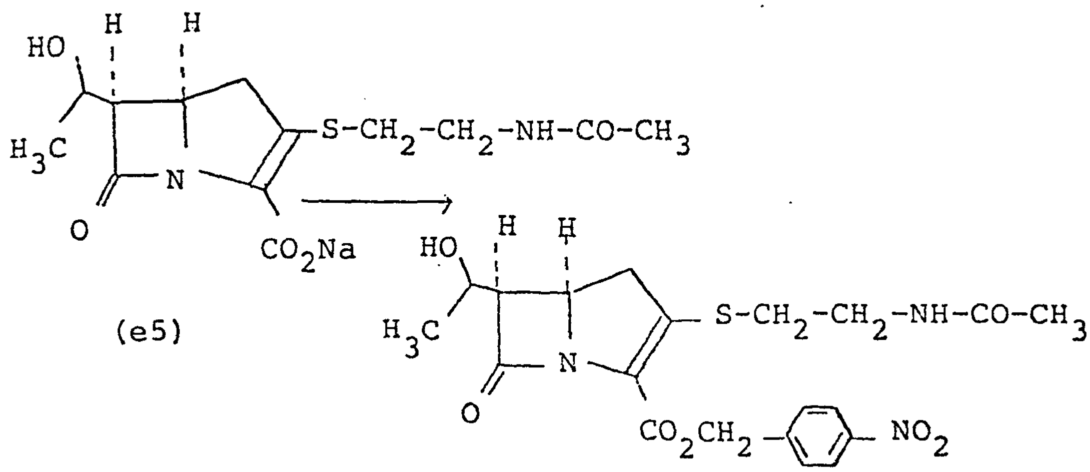

(e5)

(e6)

A crude preparation of the sodium salt (e5) (3g, about 20% pure) was suspended in dimethylformamide (35ml) and p-nitrobenzyl bromide (2g) was added with stirring. After 1.5 hours at room temperature the mixture was concentrated to small volume in vacuo and the residue diluted with ethyl acetate (100ml). The mixture was filtered over Celite and the solution washed with brine, five times with water and finally with brine again. The organic layer was dried ($MgSO_4$) and evaporated to afford a gum. This was chromatographed on silica gel (50g) using ethyl acetate/ethanol (4/1) as eluent. The required fractions (tlc) were combined and evaporated to give a yellow solid. Further chromatography on silica gel (30g) using gradient elution ($CHCl_3 \longrightarrow CHCl_3:C_2H_5OH$, 3:1) afforded the purified ester as a white solid. Recrystallisation from ethanol yielded the ester (e6) as crystalline needles.

m.p. 166-168$^{o}$ (needles from EtOH)

Found: C, 53.2; H, 5.0; N, 9.2% $C_{20}H_{23}N_3O_7S$ requires C, 53.4; H, 5.2; N, 9.4%

λ max (EtOH) 319 (13,000) and 269 nm (12,000)

ν max (KBr) 1775, 1675, 1655 and 1605 (w) $cm^{-1}$.

δ ($DMF-d_7$) 1.30 (3H, d, *J* 6Hz), 1.87 (3H, s), *ca.* 3.05 (2H, m) *ca* 3.40 (4H, m), 3.58 (1H, dd, *J* 5.5 and 10Hz) *ca.* 4.05 (1H, m) 4.30 (1H, dt, J 5.5 and 9Hz), 5.05 (1H, br, disappears with $D_2O$), 5.28 and 5.52 (each 1H, d, *J* 14Hz), 7.75 and 8.21 (each 2H, d, J 9Hz), *ca* 8.1 (1H, br, disappears with $D_2O$).

Example 4

p-Nitrobenzyl (5R,6R)-3-[(E)-2-acetamidoethenylthio]-6-[(S)-1-hydroxyethyl]-7-oxo-1-azabicyclo[3.2.0]hept-2-ene-2-carboxylate

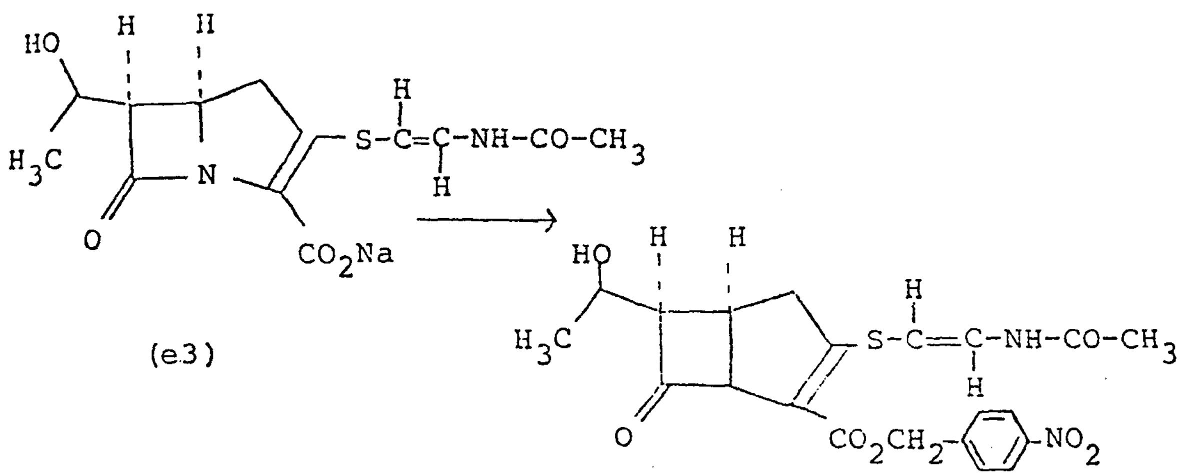

(e3)

(e7)

Using the process of Example 1 replacing the compound of the formula (e5) with the compound of the formula (e3) yielded the ester of the formula (e7) in the form of crystalline needles.

m.p. 175°-177° (needles from ethanol)
Found: C, 53.8: H, 4.4; N, 9.1% $C_{21}H_2N_3O_7S$ requires C, 53.7; H, 4.7; N, 9.4%
λ max (EtOH) 327 (16,400) and 268 (17,300) and 221 nm (18,200)
ν max (KBr) 1770, 1690sh, 1675 and 1620$cm^{-1}$
δ ($DMF-d_7$) 1.29 (3H, d, J 6Hz), 1.99 (3H, s), ca 3.25 (2H, m), 3.56 (1H, dd, J 6 and 9.5Hz), 3.95-4.45 (2H, m), 4.99 (1H, d, *J* 5.5Hz), 5.29 and 5.52 (each 1H, d, J 14Hz), 5.99 (1H, d, *J* 13.5Hz) 7.16 (1H, dd, *J* 13.5 and 10Hz), 7.76 and 8.21 (each 2H, d, *J* 9Hz)

The compounds of Example 4 was found to have MIC (PST agar + 5% Horse blood, Inoculum $10^{-4}$ dilution) of less than or equal to 2µg/ml against strains of *Staphylococcus aureus*, *Proteus mirabilis*, *Protus morganii* and *Bacillus subtilis*.

Example 5

Phthalidyl (5R,6S)-3-(2-acetamidoethylthio)-6-[(S)-1-hydroxyethyl]-7-oxo-1-azabicyclo[3.2.0]hept-2-ene-2-carboxylate

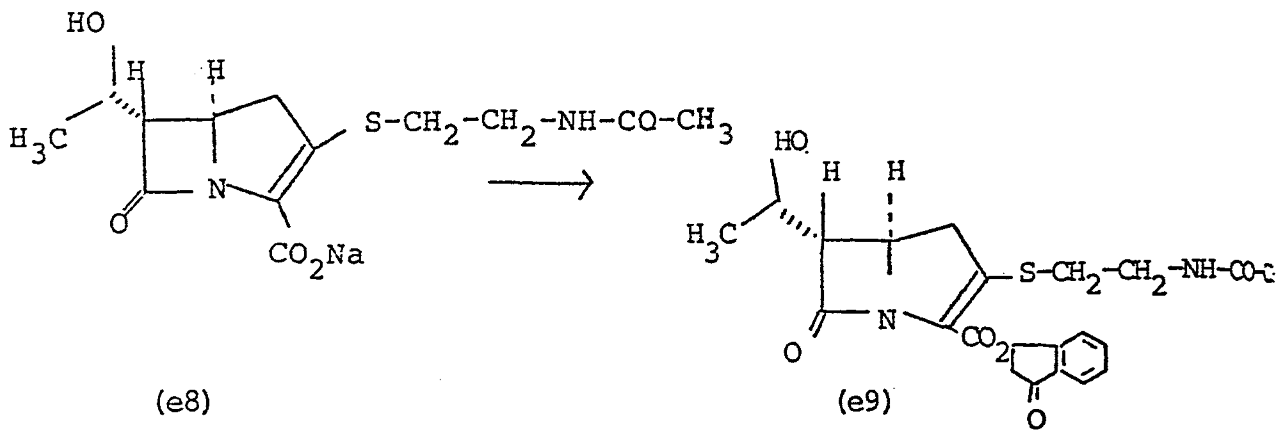

(e8) (e9)

A crude preparation of the salt (e8) (ca 40%) (0.5g) was suspended in dimethylformamide (5 ml), and to the mixture was added phthalidyl bromide (0.165g). The mixture was stirred for 1.5h at room temperature and was then concentrated in vacuo.

The residue was chromatographed on silica gel using chloroform/ethanol mixtures to elute, beginning with chloroform and finishing with 30% ethanol in chloroform. Fractions containing the product (t.l.c) were combined and concentrated in vacuo to afford the phthalidyl ester (e9) as a white amorphous solid (0.101g);

λ max. (EtOH) 327 and 229nm; ν max ($CHCl_3$) 1785, 1715 and 1665 $cm^{-1}$.

Example 6

Methyl (5R,6R)-3-[(E)-2-acetamidoethenylthio]-6-[(S)-1-hydroxyethyl]-7-oxo-1-azabicyclo[3.2.0]hept-2-ene-2-carboxylate

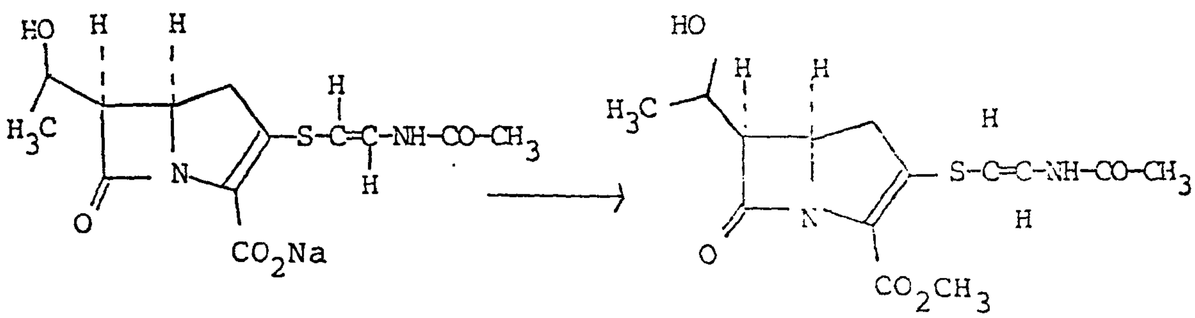

(e3) (e10)

A crude preparation of the salt (e3) (<20%) (1g) was stirred with methyl iodide (1 ml) in dimethylformamide for 1.5h at room temperature. The mixture was filtered over celite and the filtrate diluted with ethyl acetate (50 ml). The organic solution was washed with water (4 x 50ml) and brine (30 ml), and then dried ($MgSO_4$) and concentrated in vacuo.

The residue was chromatographed on silica gel using chloroform followed by 10% and then 20% ethanol in chloroform to elute. The desired ester (e10) was obtained as a white solid (33 mg);

$\lambda$ max (EtOH) 322 and 230 nm. $\nu$ max ($CHCl_3$) 1780, 1700 and 1630 $cm^{-1}$.

Example 7

Allyl (5R,6R)-3-(2-Acetamidoethylthio)-6-[(S)-1-hydroxy-ethyl]-7-oxo-1-azabicyclo[3.2.0]hept-2-ene-2-carboxylate

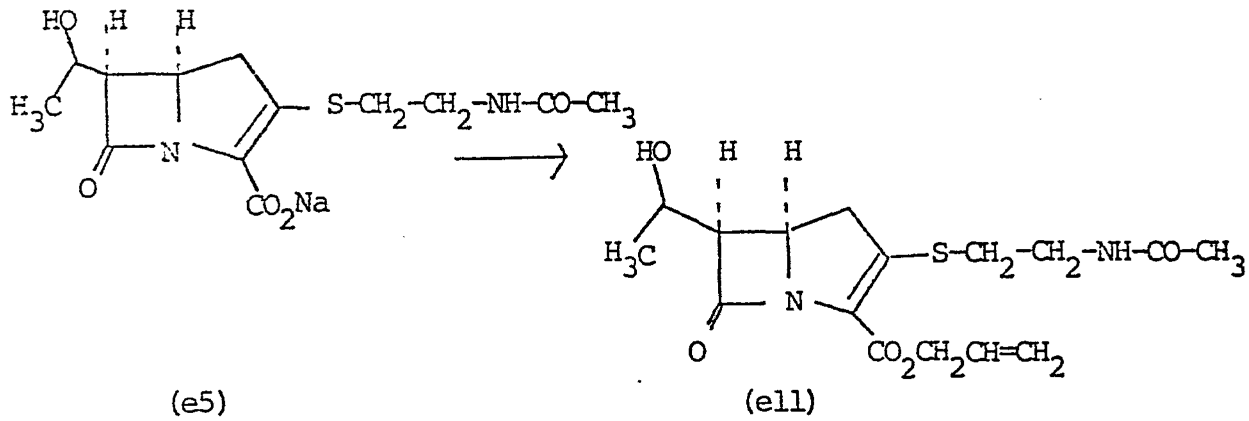

(e5) (e11)

A crude preparation of the sodium salt (e5) (50% pure, 400 mg) was suspended in  -dimethylformamide (7 ml) and treated with allyl bromide (0.8 ml). After stirring for 3½ hrs. ethyl acetate (100ml) and water (50 ml) were added to the previously filtered dimethylformamide solution. The ethyl acetate layer was washed with water (50 ml), dried ($MgSO_4$) and evaporated in vacuo to leave a gum. This was chromatographed on silica gel (Merck 9385; about 15g), eluting with $CHCl_3$ and then with $CHCl_3$/EtOH mixtures 9:1 then 8:2.

The fractions containing the ester were combined and evaporated in vacuo to give the allyl ester (e11) (63 mg), $\lambda$ max (EtOH) 314 ($\epsilon$ max 10,400) nm. $\nu$ max ($CH_2Cl_2$) 1780, 1700, 1675 $cm^{-1}$. $\delta$ $[(CD_3)_2CO]$ 1.32 (3H, d, J 6Hz, $C\underline{H}_3CH$) 1.90 (3H, s, $CH_3CO$), 2.8-3.8 (7H, m), 3.9-4.5 (3H, m, 1H exch $D_2O$), 4.5-4.9 (2H, m, $C\underline{H}_2CH=$), 5.1-5.7 (2H, m, $-CH=C\underline{H}_2$), 5.7-6.2 (1H, m, $CH_2C\underline{H}=CH_2$), 7.5 (1H, broad s, exch $D_2O$).

Example 8

Phthalidyl (5R,6R)-3-(2-acetamidoethylthio)-6-[(S)-1-hydroxyethyl]-7-oxo-1-azabicyclo[3.2.0]hept-2-ene-2-carboxylate

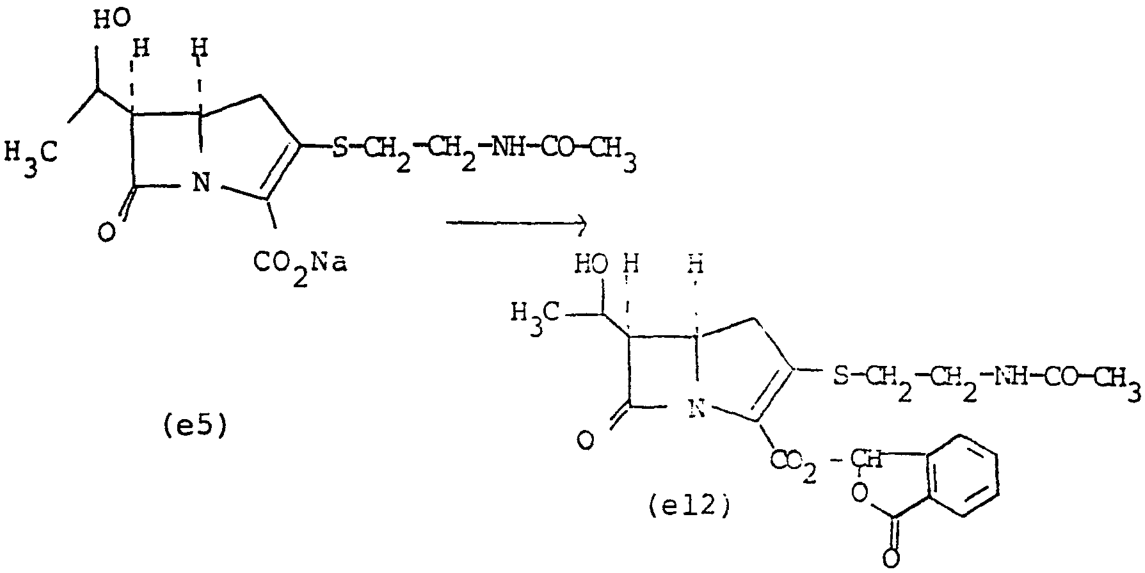

(e5)

(e12)

A crude preparation of the sodium salt (e5) (50% pure, 300 mg) in dimethylformamide (5 ml) was treated with 3-bromophthalide (150 mg) and the mixture was stirred at ambient temperature for 2hr. The dimethylformamide mixture was removed by evaporation in vacuo, the residue was suspended in chloroform and loaded on to a silica gel column (Merck 9385; about 15g). The column was eluted with chloroform and then with chloroform/ethanol mixtures; 9:1 (50 ml) and then 8:2. Combination evaporation in vacuo of the requisite fractions yielded the ester (e12) (116 mg); λ max (EtOH) 325 (9,730) and 229 (11,760) nm. ν max ($CH_2Cl_2$) 1790, 1720, 1625, 975 $cm^{-1}$.

CLAIMS

1. A compound of the formula (V) - (X):

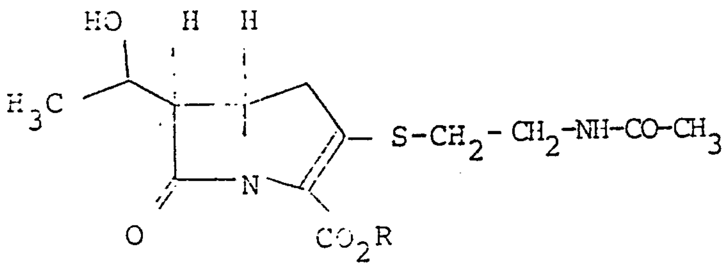

(V)

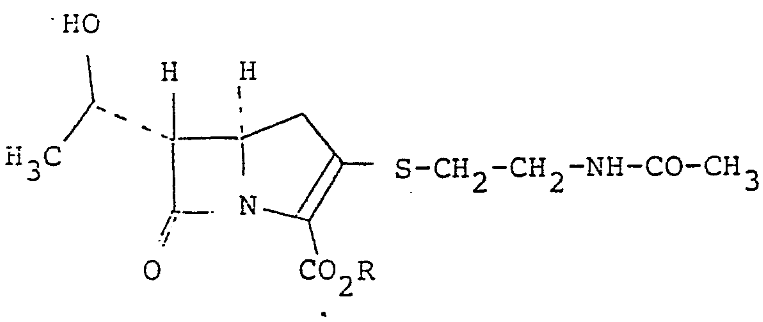

(VI)

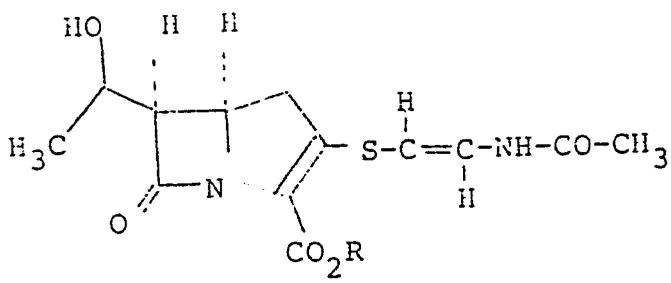

(VII)

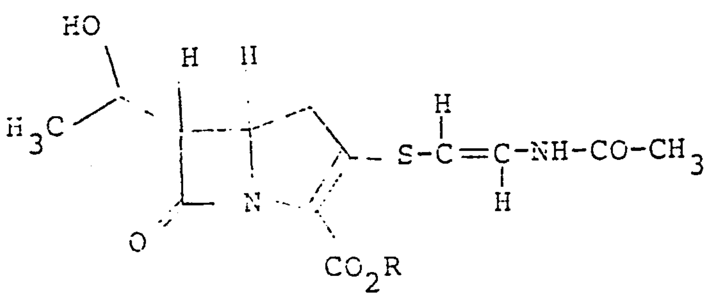

(VIII)

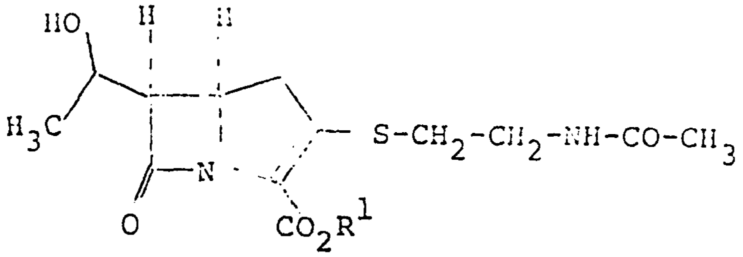

(IX)

BAD ORIGINAL

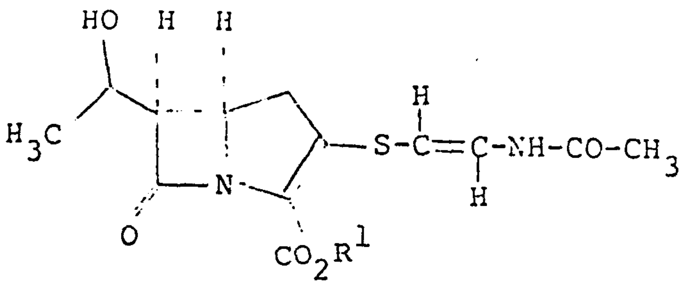

(X)

wherein R is a group of the sub-formula (a), (b) or (c):

$-CHR_1-O-CO-R_2$ (a)

$-CHR_1-O-CO-OR_2$ (b)

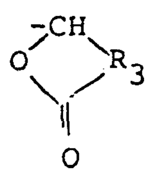

(c)

where $R_1$ is a hydrogen atom or a methyl or ethyl group; $R_2$ is a lower alkyl, lower-alkylaryl or aryl group; and $R_3$ is a $-CHR_4-CHR_4$ or $-CR_5=CR_5$ group where $R_4$ is a hydrogen atom or a methyl group and $R_5$ is a hydrogen atom, a methyl group or $R_4$ and $R_5$ together with the carbon atoms to which they are attached form the residue of a phenyl group or of a phenyl group substituted by one or two lower alkyl or lower alkoxyl groups of fluorine, chlorine or bromine atoms; and $R^1$ is a lower alkyl, lower alkenyl, lower alkynyl or aralkyl group.

2. A compound as claimed in claim 1 of the formula (v).

3. A compound as claimed in claim 1 of the formula (VII).

4. A compound as claimed in claim 1 of the formula (IX).

5. A compound as claimed in claim 1 of the formula (X).

6. A compound as claimed in any of claims 1-3 wherein $R_1$ is a hydrogen atom or a methyl group or $R_3$ is the residue of a phenyl ring.

7. A compound as claimed in any of claims 1-3 wherein R is an acetoxymethyl, pivaloyloxymethyl, α-ethoxycarbonyloxyethyl or phthalidyl group.

8. A compound as claimed in claims 4 or 5 wherein $R^1$ is a phthalidyl group.

9. A process for the preparation of a compound as claimed in claim 1 which comprises the reaction of a salt of a compound of the formula (I), (II), (III) or (IV) with a compound of the formula (XI), (XII), (XIII) or (XIV):

$Y\text{-}CHR_1\text{-}O\text{-}CO\text{-}R_2$ (XI)

$Y\text{-}CHR_1\text{-}O\text{-}CO\text{-}OR_2$ (XII)

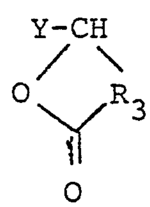 (XIII)

$Y\text{-}R^1$ (XIV)

wherein $R_1$, $R_2$ and $R_3$ are as defined in relation to sub-formulae (a), (b) and (c), $R^1$ is as defined in relation to formulae (IX) and (X) and Y is a group readily displaceable by a nucleophile.

10. A pharmaceutical composition which comprises a compound as claimed in any of claims 1-8 and a pharmaceutically acceptable carrier.
11. A compound as claimed in any of claims 1-8 for use as an antibacterial agent.

0004132

Application number

European Patent Office

# EUROPEAN SEARCH REPORT

EP 79 30 0218

## DOCUMENTS CONSIDERED TO BE RELEVANT

| Category | Citation of document with indication, where appropriate, of relevant passages | Relevant to claim |
|---|---|---|
| X | DE - A - 2 652 674 (MERCK)<br>* Claims 2,3,8, 12-19 *<br>-- | 1,2,4,<br>6-11 |
| A | DE - A - 2 736 482 (BEECHAM)<br>* Claims *<br>---- | 1-11 |

CLASSIFICATION OF THE APPLICATION (Int. Cl.[2])

C 07 D 487/04
A 61 K 31/40//
(C 07 D 487/04
209/00
205/00

TECHNICAL FIELDS SEARCHED (Int.Cl.[2])

C 07 D 487/04
A 61 K 31/40

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

p The present search report has been drawn up for all claims

| Place of search | Date of completion of the search | Examiner |
|---|---|---|
| The Hague | 12-04-1979 | CHOULY |